# EUROPEAN PATENT SPECIFICATION

(11) **EP 3 818 987 B1**
(45) Date of publication and mention of the grant of the patent: **05.03.2025**
(21) Application number: 20205961.4
(22) Date of filing: 05.11.2020
(51) Int. Cl.: A61K 36/22, A61P 31/00, A61P 11/04, A61P 27/16

(54) **PISTACIA LENTISCUS OIL FRACTIONS AND THEIR USE IN THE TREATMENT OR PREVENTION OF ORAL CAVITY INFECTIONS AND EAR, NOSE AND THROAT INFECTIONS CAUSED BY GRAM-POSITIVE OR GRAM-NEGATIVE BACTERIA**
ÖLFRAKTIONEN VON PISTACIA LENTISCUS UND DEREN VERWENDUNG ZUR BEHANDLUNG ODER VORBEUGUNG VON DURCH GRAMPOSITIVE ODER GRAMNEGATIVE BAKTERIEN VERURSACHTEN INFEKTIONEN DER MUNDHÖHLE UND HALS-NASEN-OHREN-INFEKTIONEN
FRACTIONS D'HUILE DE LENTISQUE PISTACHIER ET LEUR UTILISATION DANS LE TRAITEMENT OU LA PRÉVENTION DES INFECTIONS DE LA CAVITÉ BUCCALE ET DES INFECTIONS DES OREILLES, DU NEZ ET DE LA GORGE CAUSÉES PAR DES BACTÉRIES GRAM-POSITIVES OU GRAM-NÉGATIVES

(30) Priority: 08.11.2019 IT 201900020654
(43) Date of publication of application: 12.05.2021
(73) Proprietor: Velleja Research S.r.l., 29010 Pontenure (PC) (IT)
(72) Inventor: DI PIERRO, Francesco, 29010 PONTENURE (PC) (IT)
(74) Representative: Bianchetti & Minoja with Trevisan & Cuonzo IPS SRL

(56) References cited:
- FATEN MEZNI ET AL: "Phenolic profile and effect of growing area on Pistacia lentiscus seed oil", FOOD CHEMISTRY, vol. 257, 1 August 2018 (2018-08-01), NL, pages 206 - 210, XP055710645, ISSN: 0308-8146, DOI: 10.1016/j.foodchem.2018.03.019
- FATEN MEZNI ET AL: "Evaluation of Pistacia lentiscus seed oil and phenolic compounds for in vitro antiproliferative effects against BHK21 cells", PHARMACEUTICAL BIOLOGY, vol. 54, no. 5, 6 October 2015 (2015-10-06), NL, pages 747 - 751, XP055710648, ISSN: 1388-0209, DOI: 10.3109/13880209.2015.1079222
- F. MEZNI ET AL: "In vitro antimicrobial activity of Pistacia lentiscus L. edible oil and phenolic extract", NATURAL PRODUCT RESEARCH, vol. 29, no. 6, 26 August 2014 (2014-08-26), GB, pages 565 - 570, XP055710466, ISSN: 1478-6419, DOI: 10.1080/14786419.2014.952232
- GERMANO ORRÙ ET AL: "The Selective Interaction of Pistacia lentiscus Oil vs. Human Streptococci, an Old Functional Food Revisited with New Tools", FRONTIERS IN MICROBIOLOGY, vol. 8, 24 October 2017 (2017-10-24), XP055710468, DOI: 10.3389/fmicb.2017.02067

## Description

The present invention relates to fractions of *Pistacia lentiscus* edible oil enriched with polyphenols, obtainable by a process comprising ultracentrifugation followed by winterisation of the supernatant, solubilisation in water-alcohol mixtures, sonication and removal of the alcohol-based solvent.

The fractions according to the invention have bactericidal action against Gram-negative oral pathogenic bacteria, in particular against *Moraxella catarrhalis* and *Haemophilus influenzae.*

The fractions according to the invention produce significant synergic effects when used in combination with antibiotics, in particular with beta-lactam antibiotics, effectively eradicating bacterial infections caused by both Gram-negative bacteria (*Moraxella catarrhalis, Haemophilus influenzae*) and Gram-positive bacteria (*Streptococcus pyogenes* and *Streptococcus pneumoniae*)*.*

### Prior art

The current emergency relating to the increasingly widespread antibiotic resistance of many species of pathogenic bacteria poses a serious health problem. In an attempt to extend research designed to solve said problem, the scientific community has focused on new treatment strategies, and recently expressed renewed interest in plant-based compounds (Pogány Simonová M, et al., Res Vet Sci. 2010 Apr;88(2):203-7; Verkaik MJ et al., J Dent. 2011 Mar; 39(3):218-24). The antibiotic potential of most of said derivatives, used for "non-antibiotic" applications, is little known (Reid G. Curr Infect Dis Rep. 2000 Dec;2(6):518-522.). Antibiotic resistance is due (*inter alia*) to the ability of many bacteria to forma biofilms that provide a protective waterproof coating for antimicrobials. Biofilm formation has been described for various human body tissues, for example in the oral cavity and on the skin (Flemmig TF, Beikler T. Periodontol 2000. 2011 Feb;55(1):9-15; Vieira Colombo AP et al., Microb Pathog. 2016 May;94:27-34).

One solution to this problem could therefore be the development of active ingredients able to damage the biofilm that protects the bacterial colony. Recent observations (Orrù G, Front Microbiol. 2017 Oct 24;8:2067) demonstrate that the edible oil of the fruit of *Pistacia lentiscus,* a plant used in traditional medicine (Bozorgi M et al., Scientific World Journal. 2013 Dec 15;2013:219815), exhibits antibacterial activity against Gram-positive bacteria such as *Streptococcus agalactiae, Streptococcus intermedius, Streptococcus mitis, Streptococcus mutans* and *Streptococcus pyogenes.* Other authors (Mezni F et al., Nat Prod Res. 2015;29(6):565-70) have reported its antibiotic action against *Staphylococcus aureus* and its anti-fungal action against *Candida* spp. and *Aspergillus niger.* The majority of the research conducted in the past focused on the antimicrobial properties of the inedible part, i.e. the mastic gum and essential oil, obtained from the resin and the branches/leaves respectively. Mastic gum (produced on the Greek island of Chios) possesses proven antibacterial activity against *Helicobacter pylori,* while the derivatives formulated from the essential oil have proved active against oral pathogens such as *Porphyromonas gingivalis, Streptococcus mutans, Streptococcus gordonii, Fusobacterium nucleatum* and *Prevotella intermedia* (Paraschos S et al., Curr Med Chem. 2012;19(14):2292-302; Aksoy A et al., Arch Oral Biol. 2006 Jun;51(6):476-81; Liu W et al., Zhongguo Zhong Yao Za Zhi. 2019 Sep;44(17):3684-3694).

The medical literature therefore demonstrates that the edible oil is primarily active against Gram-positive species, and only the essential oil, which is inedible, also exhibits some activity against Gram-negative species such as *Prevotella* and *Fusobacterium,* which are involved in periodontal disease and gingivitis.

Healthy oral microbiota (Abranches J et al., Microbiol Spectr. 2018 Oct;6(5)) contain a predominance of *Streptococcus salivarius,* which can constitute up to 80% of the entire bacterial flora. However, dysbiosis of the oral cavity promotes the growth of pathogenic species liable to cause pharyngotonsillitis and otitis media. The pathogens most frequently involved are *Streptococcus pyogenes and Streptococcus pneumoniae* (Gram-positive), *Moraxella catarrhalis* and *Haemophilus influenzae* (Gram-negative). Edible mastic (or lentisk) oil only performs an antibacterial action against *Streptococcus pyogenes,* and is wholly ineffective against other species. Moreover, the action of edible mastic oil has no synergic effects when administered simultaneously with antibiotics.

In view of the absence of a *Moraxella* vaccine and the aggressive nature of *Haemophilus,* the only vaccine for which is effective against only one of the six existing serotypes (serotype B), said two Gram-negative species are of great pathological importance, especially in the paediatric field.

### Description of the invention

It has now been found that when *Pistacia lentiscus* edible oil undergoes a process comprising an ultracentrifugation step followed by a supernatant winterisation step, solubilisation in water-alcohol mixtures, preferably water-ethanol mixtures, sonication and removal of the water-alcohol solvent, a fraction enriched in phenol compounds with advantageous antibacterial properties is obtained.

According to the invention, the ultracentrifugation is preferably conducted at 15,000-20,000 rpm for 10-20 minutes.

Winterisation is a fractionated crystallisation conducted on edible fats, which is designed to separate a plurality of fractions with different melting points. In the treatment of olive oil, for example, winterisation is conducted during rectification, after deodorising, and consists of fast cooling to a temperature of about 5-10°C, kept constant for several (6-12) hours. The triglycerides with the lowest degree of unsaturation thus crystallise, making separation by filtration possible. The elimination of said substances prevents the oil from clouding at cold temperatures and makes it less viscous. The excess waxes, if any, are separated at the same time.

According to the invention, the *Pistacia lentiscus* oil is winterised by treatment at a temperature ranging between -50°C and -90°C, preferably at -80°C, for a time ranging between 24 and 48 hours, preferably 36 hours. The fixed oil derived as supernatant is then mixed with an ethanol/water mixture (75/25; v/v). Specifically, 100 g of oil is mixed with 250 ml of water-ethanol solution for 10 minutes in a vortex. The mixture is then isolated in an ultrasonic bath for 30 minutes at room temperature and centrifuged at 15000 rpm for 10 minutes. The water-ethanol phase is then removed by drying, stored in a cold, dark place and mixed again at the ratio of 4:1 with the oil obtained after winterisation. The total polyphenol content of the final fractions thus obtained ranges between 3 and 6% by weight.

The fractions according to the invention, in the form of a straw-coloured liquid, have an antimicrobial action, which is also observed at ng/mL concentrations, against *Moraxella catarrhalis* and *Haemophilus influenzae.*

The fractions according to the invention are particularly useful in the treatment or prevention of oral cavity infections and ear, nose and throat infections caused by Gram-positive or Gram-negative bacteria, in particular those caused by *Moraxella catarrhalis* or *Haemophilus influenzae.*

The fractions according to the invention are particularly indicated for adjuvant treatment, concomitantly with antibiotic treatment, of pharyngotonsillitis and otitis media, especially if they are resistant to conventional antibiotic treatments.

It has been observed that the fractions according to the invention produce synergic effects with many conventional antibiotics. Such synergy can be observed in the culture plate, wherein the action of the mastic oil fraction induces the death of 100% of colonies of *S. pneumoniae, S. pyogenes, M. catarrhalis* and *H. influenzae,* in the presence of 10, 10, 15 and 20% respectively of the antibiotic required to obtain the same result. Said performance has been demonstrated using amoxicillin and clavulanic acid, amoxicillin alone, and 1st, 2nd, 3rd and 4th generation cephalosporins.

As observed in BHI-Congo red plates, the action is mediated by total destruction of the biofilms that the *Moraxella catarrhalis* and *Haemophilus influenzae* strains can still produce.

The edible mastic oil fractions thus obtained, hereinafter called "OLUW", can be solubilised as a macerated oil in triglycerides and administered in the form of drops, or formulated after adsorption as chewable tablets or orosoluble sachets.

The invention is illustrated in detail in the examples below.

### EXAMPLE 1 - Gum disc

| **Compound** | **Quantity** |
|---|---|
| OLUW | 100 µg |
| Gum base | 200 mg |
| Levilite | 20 mg |
| Talc F.U. | 20 mg |
| Vegetable magnesium stearate | 18 mg |
| Shellac | 12 mg |
| Xylitol | 250 mg |
| Gum Arabic | 6 mg |
| Titanium dioxide | 6 mg |
| Carnauba wax | 0,2 mg |

### EXAMPLE 2 - Orodispersible formulation

| **Compound** | **Quantity** |
|---|---|
| OLUW | 100 µg |
| Sorbitol | 160 mg |
| Orange flavouring | 20 mg |
| Mandarin flavouring | 5 mg |
| Acesulfame K | 2 mg |
| Aerosil | 5 mg |
| Fructose | 1566 mg |

### Example 3 - Paediatric drops

| **Compound** | **Quantity** |
|---|---|
| OLUW | 50 µg |
| Edible triglycerides | 2,5 mg |
| Vitamin E | 1,5 mg |
| Dispersing agent/emulsifier | q.s. |

## Claims

1. Fraction of edible *Pistacia lentiscus* oil enriched in polyphenols obtainable by ultracentrifugation followed by winterisation of the supernatant, solubilisation in water-alcohol mixtures, preferably water-ethanol mixtures, sonication and removal of the water-alcohol solvent.

2. Fraction according to claim 1 wherein the ultracentrifugation is carried out at 15,000-20,000 rpm for 10-20 minutes.

3. Fraction according to claim 1 or 2, wherein the winterisation is carried out by treatment at a temperature ranging between -50°C and -90°C, preferably at -80°C, for a time ranging between 24 and 48 hours, preferably 36 hours.

4. Fraction according to one of claims 1 to 3 having a polyphenol content ranging between 3 and 6% by weight.

5. Fraction of claims 1-4 for use in the treatment or prevention of oral cavity and ear, nose and throat infections caused by Gram-positive or Gram-negative bacteria.

6. Fraction for use according to claim 5 wherein the infections are caused by *Moraxella catarrhalis* or *Haemophilus influenzae.*

7. Fraction for use according to claim 5 or 6 for the prevention of bacterial pharyngotonsillitis and acute otitis media and for the adjuvant treatment, in combination with antibiotic treatment, of pharyngotonsillitis and otitis media, especially if resistant to conventional antibiotic treatments.

8. Process for the preparation of the fractions of claims 1-4 which includes ultracentrifugation of *Pistacia lentiscus* oil followed by winterisation of the supernatant, solubilisation in water-alcohol mixtures, preferably water-ethanol mixtures, sonication and removal of the water-alcohol solvent.

9. Process according to claim 8 wherein the ultracentrifugation is carried out at 15,000-20,000 rpm for 10-20 minutes and the winterisation is carried out by treatment at a temperature ranging between -50°C and -90°C, preferably at - 80°C, for a time ranging between 24 and 48 hours, preferably 36 hours.

## Patentansprüche

1. Mit Polyphenolen angereicherte Fraktion des Speiseöls von *Pistacia lentiscus,* erhältlich durch Ultrazentrifugation und anschließende Winterisierung des Überstands, Solubilisierung in Wasser-Alkohol-Gemischen, vorzugsweise Wasser-Ethanol-Gemischen, Ultraschallbehandlung und Entfernung des Wasser-Alkohol-Lösungsmittels.

2. Fraktion nach Anspruch 1, wobei die Ultrazentrifugation bei 15.000-20.000 U/min für 10-20 Minuten durchgeführt wird.

3. Fraktion nach Anspruch 1 oder 2, wobei die Winterisierung durch Behandlung bei einer Temperatur zwischen -50°C und - 90°C, vorzugsweise bei -80°C, für eine Zeit zwischen 24 und 48 Stunden, vorzugsweise 36 Stunden, erfolgt.

4. Fraktion nach einem der Ansprüche 1 bis 3 mit einem Polyphenolgehalt zwischen 3 und 6 Gew.-%.

5. Fraktion nach Ansprüchen 1-4 zur Verwendung bei der Behandlung oder Vorbeugung von Infektionen der Mundhöhle und des Ohrs, der Nase und des Halses, die durch Gram-positive oder Gram-negative Bakterien verursacht werden.

6. Fraktion zur Verwendung nach Anspruch 5, wobei die Infektionen verursacht werden durch *Moraxella catarrhalis* oder *Haemophilus influenzae.*

7. Fraktion zur Verwendung nach Anspruch 5 oder 6 zur Vorbeugung von bakterieller Pharyngotonsillitis und akuter Otitis media und zur adjuvanten Behandlung, in Kombination mit Antibiotika-Behandlung, von Pharyngotonsillitis und Otitis media, insbesondere wenn diese gegen herkömmliche antibiotische Behandlungen resistent sind.

8. Verfahren zur Herstellung der Fraktionen nach den Ansprüchen 1-4, umfassend die Ultrazentrifugation von *Pistacia-lentiscus-*Öl*,* gefolgt von Winterisierung des Überstandes, Solubilisierung in Wasser-Alkohol-Gemischen, vorzugsweise Wasser-Ethanol-Gemischen, Ultraschallbehandlung und Entfernung des Wasser-Alkohol-Lösungsmittels.

9. Verfahren nach Anspruch 8, wobei die Ultrazentrifugation bei 15.000-20.000 U/min für 10-20 Minuten durchgeführt wird und die Winterisierung durch Behandlung bei einer Temperatur zwischen -50°C und -90°C, vorzugsweise bei -80°C, für eine Zeit zwischen 24 und 48 Stunden, vorzugsweise 36 Stunden, durchgeführt wird.

## Revendications

1. Fraction d'une huile alimentaire de *Pistacia lentiscus* enrichie en polyphénols, que l'on peut obtenir par ultracentrifugation, suivie d'une wintérisation du produit surnageant, d'une solubilisation dans des mélanges eau-alcool, de préférence des mélanges eau-éthanol, d'une sonication et d'une élimination du solvant à base d'eau-alcool.

2. Fraction selon la revendication 1, dans laquelle l'ultracentrifugation est mise en œuvre à 15.000 - 20.000 tours par minute pendant 10 à 20 minutes.

3. Fraction selon la revendication 1 ou 2, dans laquelle la wintérisation est mise en œuvre par traitement à une température se situant entre -50 °C et -90 °C, de préférence à -80 °C, pendant un laps de temps se situant entre 24 et 48 heures, de préférence pendant 36 heures.

4. Fraction selon l'une quelconque des revendications 1 à 3, possédant une teneur en polyphénol se situant entre 3 et 6 % en poids.

5. Fraction selon les revendications 1 à 4, pour son utilisation dans le traitement ou la prévention d'infections de la cavité buccale et des oreilles, du nez et de la gorge dues à une bactérie Gram positif ou Gram négatif.

6. Fraction pour son utilisation selon la revendication 5, dans laquelle les infections sont dues à *Moraxella catarrhalis* ou *Haemophilus influenzae.*

7. Fraction pour son utilisation selon la revendication 5 ou 6,pour la prévention d'une pharyngo-amygdalite bactérienne et d'une otite moyenne aigüe, et pour le traitement par adjuvant, en combinaison avec un traitement antibiotique, d'une pharyngo-amygdalite et d'une otite moyenne, en particulier en cas de résistance à des traitements antibiotiques conventionnels.

8. Procédé destiné à la préparation des fractions selon les revendications 1 à 4, qui comprend une ultracentrifugation d'une huile de *Pistacia lentiscus,* suivie d'une wintérisation du produit surnageant, d'une solubilisation dans des mélanges eau-alcool, de préférence des mélanges eau-éthanol, d'une sonication et d'une élimination du solvant à base d'eau-alcool.

9. Procédé selon la revendication 8, dans lequel l'ultracentrifugation est mise en œuvre à 15.000 - 20.000 tours par minute pendant 10 à 20 minutes et la wintérisation est mise en œuvre par traitement à une température se situant entre -50 °C et -90 °C, de préférence à -80 °C, pendant un laps de temps se situant entre 24 et 48 heures, de préférence pendant 36 heures.
